# EUROPEAN PATENT SPECIFICATION

(11) **EP 3 441 057 B1**
(45) Date of publication and mention of the grant of the patent: **23.06.2021**
(21) Application number: 18788990.2
(22) Date of filing: 17.01.2018
(51) Int. Cl.: A61H 7/00

(54) **MASSAGE MOTION DEVICE AND HEAD SCRATCHING MASSAGER**
MASSAGEBEWEGUNGSVORRICHTUNG UND KOPFKRATZMASSAGEGERÄT
DISPOSITIF DE MOUVEMENT DE MASSAGE ET DISPOSITIF DE MASSAGE POUR GRATTAGE DE TÊTE

(30) Priority: 16.06.2017 CN 201710459381
(43) Date of publication of application: 13.02.2019
(73) Proprietor: Shenzhen Breo Technology Co., Ltd., Shenzhen, Guangdong 518000 (CN)
(72) Inventor: MA, Xuejun, Shenzhen Guangdong 518000 (CN); LI, Rui, Shenzhen Guangdong 518000 (CN); TAO, Xianle, Shenzhen Guangdong 518000 (CN)
(74) Representative: Wilson Gunn
(86) International application number: PCT/CN2018/073028
(87) International publication number: WO 2018/227968

(56) References cited:
- CN-A- 103 470 718
- CN-A- 106 038 158
- CN-A- 107 233 199
- CN-U- 201 516 136
- CN-U- 201 516 136
- CN-U- 204 544 503
- CN-U- 205 041 730
- CN-U- 205 041 730
- CN-U- 205 251 984
- DE-U1-202016 101 281
- JP-A- 2011 125 514
- JP-U- 3 198 428

## Description

### TECHNICAL FIELD

The present application relates to the technical field of massager, and more particularly to a massage motion device and a massager for scratching head.

### BACKGROUND

The closest prior art to the present application is a Chinese patent with application No. CN205251984U and has disclosed a massage comb, including: a bottom case 30, a base plate 50, a power device 60 and a moveable comb body 40, the power device60 includes a motor 61 and a first teeth 62, the comb body 40 includes a bottom plate 41and a boss 43 arranged on the bottom plate 41, a lower end surface of the first teeth is provided with a circular groove 621,the centre of the circular groove 621 is deviated from the center of a rotation shaft of the first teeth 62, and the boss 63 is inserted into the circular groove 621.

The massage action of the existing massage motion device is simulation of rotation, scratch, press and so on, which cannot simulate the massage action of scratching of the human hand, and the massage experience is poor.

A German application DE202016101281U1 discloses a handheld malaxation massage device, which further discloses that the handheld malaxation massage device can be used for massage in a malaxation mode. DE202016101281U1 discloses a kneading massager comprising swinging arm assemblies, each comprising a rotational shaft connected to a mounting seat and a guiding pin disposed in a groove formed in a rotational wheel.

A Chinese application CN201516136U discloses a massage head driving device for imitating hand grabbing and rubbing, which further discloses that the massage heads can imitate the grabbing and rubbing of the hands by the spreading and folding action of two massage arms.

A Chinese application CN204544503U discloses a sprinkler mechanism and shower head having massage and washing function.

A Chinese application CN205041730U discloses a 3D massage core and 3D massaging machine.

### SUMMARY

The massage motion device exists the problem that it is cannot simulate the massage action of scratching of the human hand.

The present invention provides a massage motion device according to claim 1.

Optionally, the slot extends along the length direction thereof to the upper side of the connecting arm and forms a semi-opening hole.

Optionally, the number of the second rotational shaft is two, and the two second rotational shafts are connected to side arms of the swinging arm oppositely disposed; the connecting arm includes a pair of connecting branch arms interval disposed, the two connecting branch arms are respectively located at two sides of the swing arm, and any one of the connecting branch arms on a sidewall thereof facing the other connecting arm is provided with the slot, and one of the slots is matched with one of the second rotational shaft.

Optionally, each of the slots are through hole structures.

Optionally, the periphery of the rotating wheel is provided with a driven teeth, the driving assembly includes a driving wheel, and a driving device for driving the driving wheel to rotate, the driving wheel is located between the two rotating wheels oppositely disposed, and the driving wheel is abutted against the peripheries of the two rotating wheels, and the periphery of the driving wheel is provided with driving teeth mesh with the driven teeth.

Optionally, the massage motion device comprises two pairs of the swing arm assemblies, and the four swing arm assemblies are arranged in a circular array.

Optionally, the number of teeth of the driven tooth is greater than the number of teeth of the driving teeth.

Optionally, the connecting member further includes a sleeved on the guide pin, and the sleeve is rotatably matched with the guide pin.

Optionally, each of the rotating wheels is connected to the mounting seat via a third rotational shaft, and the third rotational shaft is fixed to the mounting seat.

The present application further provides a massager for scratching head including the above-described massage motion device.

According to the structure of the present application, in a specific use process, the driving assembly drives the rotating wheels to rotate synchronously, wherein the guide pin placed in the guide groove moves back and forth in the guide groove in a direction perpendicular to the second rotational shaft along the rotation of the rotating wheel, while during the movement of the guide pin, the guide pin drives the connecting arm to move back and forth in a direction perpendicular to the second rotational shaft, thereby driving the swing arm to swing around the second rotational shaft, and the swing arm assemblies are arranged in pairs, the two swing arms of the pair of swing arm assemblies will swing toward or away from each other at the same time, thereby realizing the action of scratching of the human hand, so that after the massage component is mounted on the swing arm, it can obtain well massage experience.

Since the length extending direction of the slot is parallel to the extending direction of the guide pin, the interaction force between the connecting arm and the second rotational shaft are perpendicular to the wall of the length direction of the slot, thereby preventing the connecting arm from affecting form the force parallel to the extending direction of the guide pin, that is, preventing the whole connecting member from affecting form the force parallel to the extending direction of the guide pin, thereby preventing the situation the connecting member from jumping in parallel with the extending direction of the guide pin, and enhance stability.

### BRIEF DESCRIPTION OF THE DRAWINGS

In order to make the technical solutions in the embodiments of the present application clearer, the accompanying drawings to be used in the embodiments and the description of the prior art will be briefly introduced below, it is apparent that the drawings in the following description are merely some embodiments of the present application and that other drawings may be obtained by those skilled in the field without departing from the inventive nature of the application.
FIG. 1 is a partial schematic structure view of a massage motion device provided by an embodiment of the present application;
FIG. 2 is a cross-sectional view of FIG.1 in an AA direction;
FIG. 3 is an exploded view of FIG. 1.

### Description of the reference numerals:

200 rotating wheel;
201 guide groove;
400 swing mechanism;
410 swing arm;
420 connecting member;
4211 slot;
4212 connecting branch arm;
422 guide pin;
610 first rotational shaft;
620 second rotational shaft; and
630 third rotational shaft.

### DETAILED DESCRIPTION OF THE EMBODIMENTS

In order to make the technical problems to be solved, technical solutions, and beneficial effects of the present application clearer and more understandable, the present application will be further described in detail herein after with reference to the accompanying drawings and embodiments. It should be understood that the embodiments described herein are only intended to illustrate but not to limit the present application.

The present application embodiment provides a massage motion device.

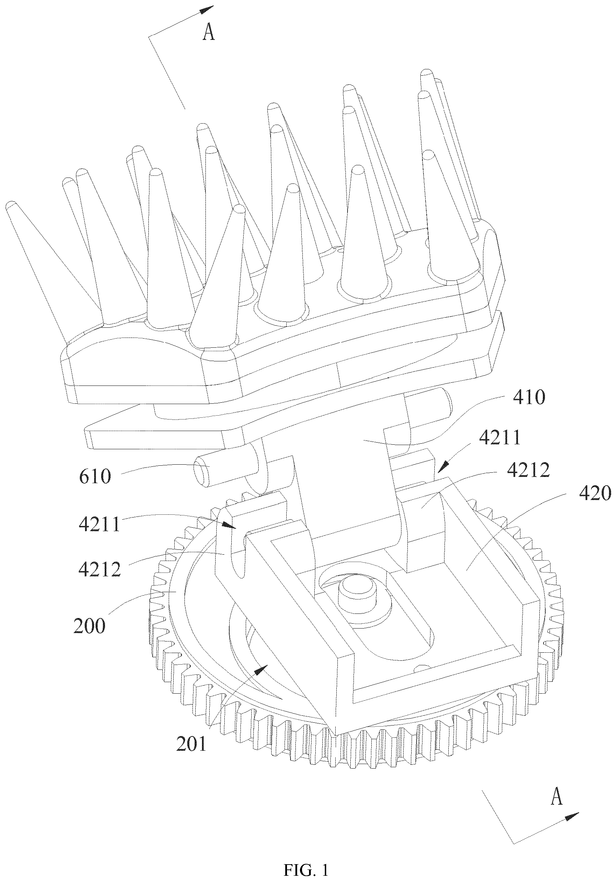

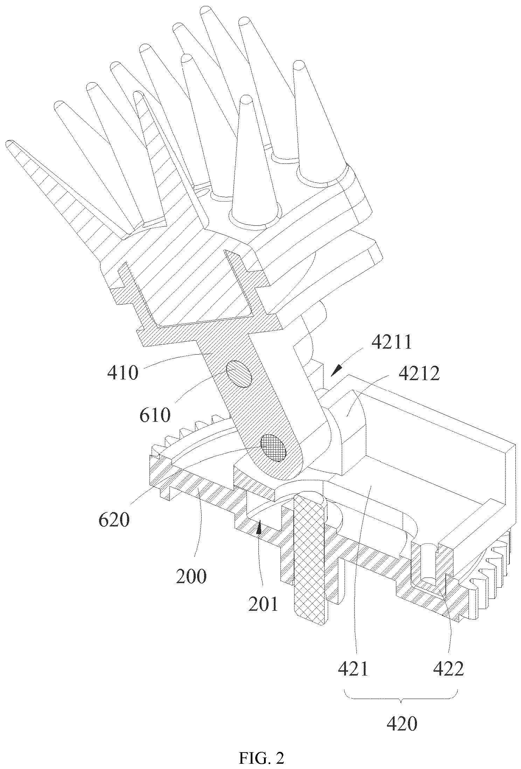

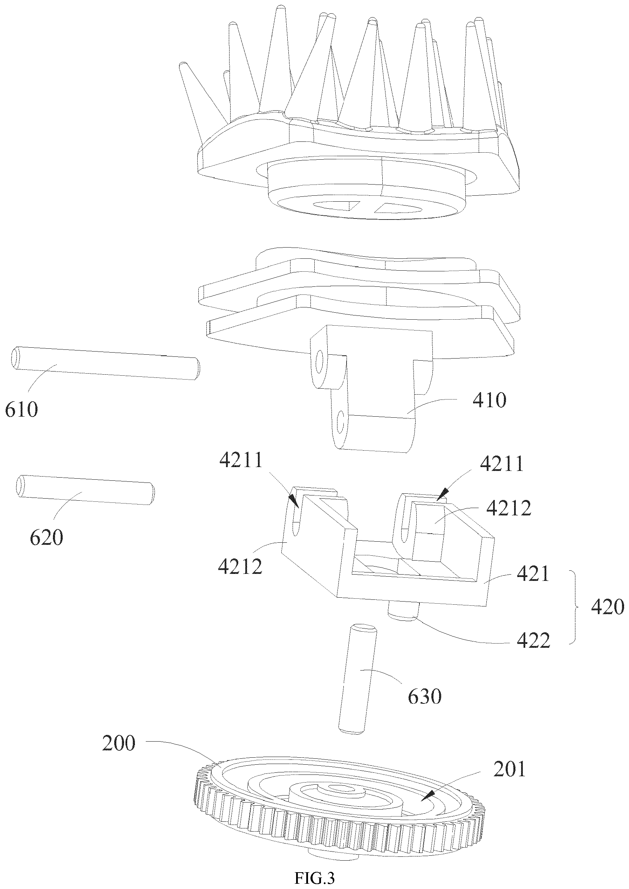

Please refer to FIGS. 1 to 3, the massage motion device includes a mounting seat and a pair of swing arm assemblies oppositely disposed; each of the swing arm assemblies being mounted to the mounting seat.
wherein each of the swing arm assemblies includes a rotating wheel and a swing arm mechanism 400, the swing arm mechanisms 400 each includes a swinging arm 410 connected to the mounting seat via a first rotational shaft 610, and a connecting member 420 rotatably connected to the swinging arm 410 via a second rotational shaft 620, and the second rotational shaft 620 is connected to a side arm of the swinging arm 410, the axis of the first rotational shaft 610 is parallel to the axis of the second rotational shaft 620.
a side surface of the rotating wheel 200 is provided with a guide groove 201, and an arrangement path of the guide groove201 is annular, and the geometric center of each of the guide grooves 210 are located in the axis of rotation of the adjacent rotating wheel 200 at a side thereof closer to or away from another the rotating wheel 200, the connecting member 420 includes a connecting arm 421 and a guide pin422 connected to the connecting arm 421 and disposed in the guide groove 201, the connecting arm 421 is provided with a slot 4211 matched with the second rotational shaft 620, the slot 4211 is disposed in a direction parallel to the axis of the second rotational shaft 620, the width of the slot 4211 is matched with the diameter of the second rotational shaft 620, and the length extending direction of the slot 4211 is disposed in parallel to the extending direction of the guide pin 422. wherein since the geometric center of each of the guide grooves 210 are located in the axis of rotation of the adjacent rotating wheel 200 at a side thereof closer to or away from another the rotating wheel 200, in this way, the two rotating wheels 200 can drive the two guide pins 422 move toward or away from each other during the synchronous rotation; in the embodiment of the present application, the arrangement path of the guide grooves 201 is annular, and the center of the annular is offset from the rotational axis of the rotating wheel 200. And since the length extending direction of the slot 4211 is parallel to the extending direction of the guide pin 422, the interaction force between the connecting arm 421 and the second rotational shaft 620 are perpendicular to the wall of the length direction of the slot 4211, thereby preventing the connecting arm 421 from affecting form the force parallel to the extending direction of the guide pin 422, that is, preventing the whole connecting member 420 from affecting form the force parallel to the extending direction of the guide pin 422, thereby preventing the situation the connecting member 420 from jumping in parallel with the extending direction of the guide pin 422, and enhance stability.

In the above, since the first rotational shaft 610 is stationary and the connecting arm 421 can only move linearly, the moving path of the connecting arm 421 is perpendicular to the first rotational shaft, so that a connection point between the connecting arm 421 and the swing arm 410 vary with the movement of the connecting arm 421, and the swing arm 410 is connected to the second rotational shaft 620 by provided the slot 4211, ensuring that the swing arm 410 can swing normally.

Please continuing refer to FIG. 1 to FIG. 3, in the embodiment of the present application, the massage motion device includes a driving assembly for driving the rotating wheels 200 to rotate synchronously.

According to the structure of the present application, since the extending direction of the guide pin 422 is perpendicular to the direction of the first rotational shaft 610, the guide pin 422 can only move in a direction perpendicular to the first rotational shaft 610, and also because the geometric center of each of the guide grooves 201 are located in the axis of rotation of the adjacent rotating wheel 200 at a side thereof closer to or away from another the rotating wheel 200, and the distance from the position where the guide pin 422 matched with the guide groove 201 to the rotational axis of the rotating wheel 200 will vary with the rotation of the rotating wheel 200.

In a specific use process, the driving assembly drives the rotating wheels 200 to rotate synchronously, wherein the guide pin 422 placed in the guide groove 201 moves back and forth in the guide groove 201 in a direction perpendicular to the second rotational shaft 620 along the rotation of the rotating wheel 200, while during the movement of the guide pin 422, the guide pin422 drives the connecting arm 421 to move back and forth in a direction perpendicular to the second rotational shaft 620, thereby driving the swing arm 410 to swing around the second rotational shaft 620, and the swing arm assemblies are arranged in pairs, the two swing arms 410 of the pair of swing arm assemblies will swing toward or away from each other at the same time, thereby realizing the action of scratching of the human hand, so that after the massage component is mounted on the swing arm 410, it can obtain well massage experience.

In addition, it should be emphasized here that if the swing arm 410 is directly connected to the rotating wheel 200, since the moving path of the connecting point of the swing arm 410 and the connecting arm 421 is an arc during the swinging of the swing arm 410, and appears a case of moving up and down, in this way, the arrangement path of the guide groove 201 needs to match with the swing arm 410, so that the arrangement path of the guide groove 201 is not in one plane, which results in the shape of the guide groove 201 being very complicated, which is inconvenient to manufacture and greatly increases the cost; while in the embodiment of the present application, the swing arm 410 is ganged with the rotating wheel 200 via the connecting member 420, and the slot 4211 is formed in the swing arm 410. Thus, the arrangement path of the guide groove 201 can be in a plane, the shape and configuration of the guide groove 201 is greatly simplified, which is convenient for manufacturing and greatly reduces the cost.

Specifically, in the embodiment of the present application, the cross section of the guide groove 201 on the rotating wheel 200 can be rectangular.

Please refer to the figures, the slot 4211 extends along the length direction to the upper side of the connecting arm 421, and forms a semi-opening hole. In this way, it is advantageous to improve the assembly efficiency between the connecting member 420 and the swing arm 410. Specifically, in the assembly process, the second rotational shaft 620 is simply inserted into the slot 4211 from the upper side of the connecting arm 421 to complete the assembly.

Please refer to the figures, the number of the second rotational shaft 620 is two, and the two second rotational shafts 620 are connected to side arms of the swinging arm 410 oppositely disposed; the connecting arm 421 includes a pair of connecting branch arms 4212 interval disposed, the two connecting branch arms 4212 are respectively located at two sides of the swing arm 410, and any one of the connecting branch arms 4212 on a sidewall thereof facing the other connecting arm 421 is provided with the slot 4211, and one of the slots 4211 is matched with one of the second rotational shaft 620. Thus, the connection support point between the swing arm 410 and the connecting arm 421 is increased, and the connection strength between the swing arm 410 and the connecting arm 421 is enhanced. In the present embodiment, the two second rotational shafts 620 are integrally formed and pass through the swing arm 410.

Further, each of the slots 4211 are through hole structures. In this way, it is convenient to observe whether the second rotational shaft 620 and the slot 4211 are assembled well during the assembly of the connecting member 420 and the swing arm 410, which is advantageous for improving assembly efficiency.

Please refer to the figures, the periphery of the rotating wheel 200 is provided with driven teeth, the driving assembly includes a driving wheel, and a driving device for driving the driving wheel to rotate, the driving wheel is located between the two rotating wheels 200 oppositely disposed, and the driving wheel is abutted against the peripheries of the two rotating wheels 200, and the periphery of the driving wheel is provided with driving teeth mesh with the driven teeth. Based on this design, only one driving wheel is needed, so that only one driving device for driving the driving wheel to rotate is needed, and it is not necessary to separately set the driving wheel and the driving device for each set of swing arm assemblies, thus reducing the driving source and beneficial to reduce manufacturing costs. Specifically, in the embodiment of the present application, the driving device is a driving motor.

In an embodiment of the present application, the massage motion device comprises two pairs of swing arm assemblies, the four swing arm assemblies being arranged in a circular array. Wherein, the added pair of swing arm assemblies also share the same driving wheel with another pair of swing arm assemblies.

Please refer to the figures, the number of teeth of the driven teeth is greater than the number of teeth of the driving teeth. Since the number of teeth of the driven teeth is greater than the number of teeth of the driving teeth, that is, when the driving teeth rotates one round, the driven teeth has not been rotated enough one round, thus the transmission ratio of the transmission component is improved, and the output torque of the driving device can be improved. Specifically, in the present embodiment, the ratio of the number of teeth of the driven teeth to the number of teeth of the driving teeth is 2:1.

Please refer to FIGS. 1 to 3, the connecting member 420 further includes a sleeve that is sleeved on the guide pin 422, and the sleeve is rotatably matched with the guide pin 422. Based on this structure, the guide pin 422 is in contact with the groove wall of the guide groove 201 via the sleeve, wherein since the sleeve is rotatably matched with the guide pin 422, so that when the outer side of the guide pin 422 and the groove wall of the guide groove 201 have a larger frictional resistance, the sleeve can be rotated relative to the guide pin 422, and the frictional resistance between the outer side of the guide pin 422 and the groove wall of the guide groove 201 can be prevented from being unable to slide in the guide groove 201.

Please refer to FIG. 1 to FIG. 3, each of the rotating wheels 200 is connected to the mounting seat via a third rotational shaft 630, and the third rotational shaft 630 is fixed to the mounting seat.

Please refer to FIG. 1 to FIG. 3, the massage motion device further includes at least two massage components (not shown), and each of the swing arms 410 is connected with one massage component. In this way, after the assembly of the massage motion device is completed, there is no need to additionally assemble the massage component.

The present application further provides a massager for scratching head, which includes the massage motion device, and the specific structure of the massage motion device is referred to the above embodiment. Since the massager for scratching head adopts all the technical solutions of all the above embodiments, Therefore, all the beneficial effects brought about by the technical solutions of the above embodiments are not repeated here.

The above are only the preferred embodiments of the present application, and are not intended to limit the present application. Any modifications, equivalent substitutions or improvements made within the scope of the claims of the present application are included in the scope of the present application.

## Claims

1. A massage motion device, comprising: a mounting seat and a pair of swing arm assemblies (400) oppositely disposed; each of the swing arm assemblies (400) being mounted to the mounting seat, each of the swing arm assemblies comprises a rotating wheel (200) and a swing arm mechanism (400),
wherein each the swing arm assemblies (400) comprises a first rotational shaft (610), a swinging arm (410) connected to the mounting seat via the first rotational shaft (610), a second rotational shaft (620) and a connecting member (420) connected to the swinging arm (410) via the second rotational shaft (620);
wherein the swinging arm (410) comprises a base portion and a top portion located at two sides of the first rotational shaft (610), and the second rotational shaft (620) is mounted at the base portion of the swinging arm (410);
wherein an axis of the first rotational shaft (610) is parallel to an axis of the second rotational shaft (620), and
wherein an upper surface of the rotating wheel (200) is provided with a guide groove (201), and an arrangement path of the guide groove (201) is annular, and the geometric center of each of the guide groove (201) is offset from a rotational axis of the rotating wheel (200), and
the connecting member (420) comprises a connecting arm (421), a guide pin (422) connected to the connecting arm (421) and disposed in the guide groove (201), and a slot (4211) disposed on the connecting arm (421) and configured to match with the second rotational shaft (620), the slot (4211) defines a pair inner wall portions disposed opposite to each other and configured to be in contact with the second rotational shaft (620); the pair inner wall portions extend in a first direction parallel to the axis of the second rotational shaft (620), and extend in a second direction parallel to an extending direction of the guide pin (422); and whereby the massage motion device comprises a driving assembly for driving the rotating wheels (200) to rotate synchronously.

2. The massage motion device of claim 1, wherein the slot (4211) extends along the second direction to an upper side of the connecting arm (421) and forms a semi-opening thereon.

3. The massage motion device of claim 1, wherein the connecting arm (421) comprises a pair of connecting branch arms (4212) disposed apart from each other, the two connecting branch arms are respectively located at two sides of the swing arm along the axis of the second rotational shaft (620), and each of the connecting branch arms (4212) is provided with the slot (4211).

4. The massage motion device of claim 3, wherein each of the slots (4211) is a through groove configured for the second rotational shaft (620) passing through.

5. The massage motion device of claim 1, wherein the periphery of the rotating wheel (200) is provided with a driven teeth, the driving assembly comprises a driving wheel, and a driving device configured for driving the driving wheel to rotate, the driving wheel is located between the two oppositely disposed rotating wheels (200), and the driving wheel is abutted against the peripheries of the two rotating wheels (200), and the periphery of the driving wheel is provided with driving teeth mesh with the driven teeth.

6. The massage motion device of claim 1, wherein the connecting member (420) further comprises a sleeve sleeved on the guide pin (422), and the sleeve is rotatably matched with the guide pin (422).

7. A massager for scratching head, comprising: the massage motion device according to any one of claims 1-6.

## Patentansprüche

1. Massagebewegungsvorrichtung, aufweisend:
einen Montagesitz und ein Paar von Schwenkarmanordnungen (400), die einander gegenüberliegend angeordnet sind; wobei jede der Schwenkarmanordnungen (400) an dem Montagsitz montiert sind, wobei jede der Schwenkarmanordnungen ein rotierendes Rad (200) und einen Schwenkarmmechanismus (400) aufweist;
wobei jeder der Schwenkarmanordnungen (400) ein erste Drehachse (610), einen Schwenkarm (410), welcher mit dem Montagesitz über die erste Drehachse (610) verbunden ist, eine zweite Drehachse (620) und ein Verbindungselement (420), welche mit dem Schwenkarm (410) über die zweite Drehachse (620) verbunden ist, aufweist;
wobei der Schwenkarm (410) einen unteren Bereich und einen oberen Bereich aufweist, die auf zwei Seiten der ersten Drehachse (610) angeordnet sind und wobei die zweite Drehachse (620) an dem unteren Bereich des Schwenkarms (410) angeordnet ist;
wobei eine Achse der ersten Drehachse (610) parallel zu einer Achse der zweiten Drehachse (620) ist; und
wobei eine ober Oberfläche des rotierenden Rades (200) mit einer Führungsnut (201) versehen ist und ein Anordnungsweg der Führungsnut (201) ringförmig ist, und der geometrische Mittelpunkt der Führungsnut (201) versetzt von einer Rotationsachse des rotierenden Rades (200) ist, und
wobei das Verbindungselement (420) einen Verbindungsarm (421), einen Verbindungsstift (422), der mit dem Verbindungsarm (421) verbunden ist und in der Führungsnut (201) angeordnet ist, und einen Schlitz (4211), der auf dem Verbindungsarm (421) angeordnet ist und konfiguriert ist, um zu der zweiten Drehachse (620) zu passen, aufweist, wobei der Schlitz (4211) ein Paar innerer Wandbereiche definiert, die gegenüber voneinander angeordnet sind und konfiguriert sind, um in Kontakt mit der zweiten Drehachse (620) zu sein; wobei das Paar der inneren Wandbereiche sich in einer ersten Richtung parallel zu der Achse der zweiten Drehachse (620) erstreckt und in einer zweiten Richtung parallel zu einer Erstreckungsrichtung des Führungsstiftes (422) erstreckt; und
wobei die Massagebewegungsvorrichtung eine Antriebsanordnung zum Antreiben der rotierenden Räder (200) zum synchronen Rotieren aufweist.

2. Massagebewegungsvorrichtung nach Anspruch 1, wobei sich der Schlitz (4311) entlang der zweiten Richtung bis zu einer oberen Seite des Verbindungsarms (421) erstreckt und ein halboffenes Loch darauf bildet.

3. Massagebewegungsvorrichtung nach Anspruch 1, wobei der Verbindungsarm (421) ein Paar von Verbindungszweigarmen (4212) aufweist, die mit Abstand zueinander angeordnet sind, wobei die beiden Verbindungszweigarme jeweils an zwei Seiten des Schwingarmes entlang der Achse der zweiten Drehachse (620) angeordnet sind und jeder der Verbindungszweiarme (4212) mit einem Schlitz (4211) versehen ist.

4. Massagebewegungsvorrichtung nach Anspruch 3, wobei jeder der Schlitze (4211) eine Durchgangsnut ist, konfiguriert, damit die zweite Drehachse (620) hindurchpasst.

5. Massagebewegungsvorrichtung nach Anspruch 1, wobei der Umfang des rotierenden Rades (200) mit einem angetriebenen Zahnrad versehen ist, wobei die Antriebseinrichtung ein Antriebsrad und eine Antriebseinrichtung aufweist, die konfiguriert ist, um das Antriebsrad zum Rotieren anzutreiben, wobei das Antriebsrad zwischen den beiden gegenüberliegend angeordneten rotierenden Rädern (200) angeordnet ist, wobei das Antriebsrad an den Umfängen der beiden rotierenden Räder (200) anliegt und der Umfang des Antriebsrades mit einem Antriebszahnrad versehen ist, das mit dem angetriebenen Zahnrad kämmt.

6. Massagebewegungsvorrichtung (1), wobei das Verbindungselement (420) des Weiteren aufweist eine Hülse, aufgenommen auf dem Führungsstift (422) und wobei die Hülse drehbar mit dem Führungsstift (422) zusammenpasst.

7. Massagegerät zum Kratzen des Kopfes aufweisend die Massagebewegungsvorrichtung nach einem der Ansprüche 1 bis 6.

## Revendications

1. Dispositif de massage comprenant un siège de montage et une paire de systèmes de bras pivotant (400) disposés à l'opposé l'un de l'autre, chacun des systèmes de bras pivotant (400) étant monté sur le siège de montage et chacun des systèmes de bras pivotant comprenant une roue rotative (200) et un mécanisme de bras pivotant (400),
dans lequel chacun des systèmes de bras pivotant (400) comprend un premier arbre de rotation (610), un bras pivotant (410) relié au siège de montage par le premier arbre de rotation (610), un second arbre de rotation (620) et un organe de liaison (420) relié au bras pivotant (410) via le second arbre de rotation (620),
dans lequel le bras pivotant (410) comprend une partie inférieure et une partie supérieure situées de part et d'autre du premier arbre de rotation (610), et le second arbre de rotation (620) est monté sur la partie inférieure du bras pivotant (410),
dans lequel un axe du premier arbre de rotation (610) est parallèle à un axe du second arbre de rotation (620) et
dans lequel une surface supérieure de la roue rotative (200) est dotée d'une rainure de guidage (201), la rainure de guidage (201) suivant un itinéraire annulaire et le centre géométrique de chaque rainure de guidage (201) étant décalé par rapport à un axe de rotation de la roue rotative (200), et
l'organe de liaison (420) comprend un bras de liaison (421), une broche de guidage (422) reliée au bras de liaison (421) et disposée dans la rainure de guidage (201) et une encoche (4211) disposée sur le bras de liaison (421) et configurée pour coïncider avec le second arbre de rotation (620), l'encoche (4211) définissant une paire de parties de paroi intérieures disposées à l'opposé l'une de l'autre et configurées pour être en contact avec le second arbre de rotation (620) ; la paire de parties de parois intérieures s'étend dans une première direction parallèle à l'axe du second arbre de rotation (620) et s'étendant dans une seconde direction parallèle à une direction d'extension de la broche de guidage (422) ;
et où le dispositif de massage comprenant un système d'entraînement pour entraîner les roues rotatives (200) pour tourner de manière synchrone.

2. Dispositif de massage selon la revendication 1, dans lequel l'encoche (4211) s'étend le long de la seconde direction sur une face supérieure du bras de liaison (421) et forme une semi-ouverture sur celle-ci.

3. Dispositif de massage selon la revendication 1, dans lequel le bras de liaison (421) comprend une paire d'embranchements de liaison (4212) disposés à distance l'un de l'autre, les deux embranchements de liaison étant situés respectivement sur deux faces du bras pivotant le long de l'axe du second arbre de rotation (620) et chacun des embranchements de liaison (4212) étant doté de l'encoche (4211).

4. Dispositif de massage selon la revendication 3, dans lequel chacune des encoches (4211) est une rainure traversante configurée pour que le second arbre de rotation (620) la traverse.

5. Dispositif de massage selon la revendication 1, dans lequel la périphérie de la roue rotative (200) est dotée d'une denture menée, le système d'entraînement comprend une roue menante et un dispositif d'entraînement configuré pour entraîner la roue menante en rotation, la roue menante est située entre les deux roues rotatives (200) disposées à l'opposé l'une de l'autre, la roue menante étant en butée contre les périphéries des deux roues rotatives (200), et la périphérie de la roue menante est dotée d'une denture menante engrenée avec la denture menée.

6. Dispositif de massage selon la revendication 1, dans lequel l'organe de liaison (420) comprend en outre un manchon emmanché sur la broche de guidage (422) et le manchon est apparié en rotation avec la broche de guidage (422).

7. Appareil de massage pour gratter la tête, comprenant le dispositif de massage selon l'une quelconque des revendications 1 à 6.
